# EUROPEAN PATENT APPLICATION

(11) **EP 4 438 130 A2**
(43) Date of publication of application: **02.10.2024**
(21) Application number: 24194793.6
(22) Date of filing: 10.07.2020
(51) Int. Cl.: A61Q 5/12

(54) **HAIR CONDITIONING COMPOSITION COMPRISING BRANCHED FATTY ALCOHOL AND TRIESTER**

(30) Priority: 11.07.2019 US 201962872840 P
(62) Divisional of application: 20751043.9
(71) Applicant: The Procter & Gamble Company, Cincinnati, OH 45202 (US)
(72) Inventor: SOH, Mui Siang, 138547 Singapore (SG)
(74) Representative: P&G Patent Germany

(57) **Abstract**

Disclosed is a hair conditioning composition comprising: a branched fatty alcohol having a melting point of below 25°C; a triester; and an aqueous carrier. The composition meets at least one of the following conditions: the composition further comprises a cationic surfactant and a high melting point fatty compound having a melting point of 25°C or more; and the weight ratio of the branched fatty alcohol to the triester is from about 9: 1 to about 1:5. The composition of the present invention provides improved hair manageability while not deteriorating other benefits especially free flowing.

## Description

### FIELD OF THE INVENTION

The present invention relates to a hair conditioning composition comprising: a branched fatty alcohol having a melting point of below 25°C; a triester; and an aqueous carrier. The composition meets at least one of the following conditions: the composition further comprises a cationic surfactant and a high melting point fatty compound having a melting point of 25°C or more; and the weight ratio of the branched fatty alcohol to the triester is from about 9:1 to about 1:5. The composition of the present invention provides improved hair manageability while not deteriorating other benefits especially free flowing.

### BACKGROUND OF THE INVENTION

A variety of approaches have been developed to condition the hair. A common method of providing conditioning benefit is through the use of conditioning agents such as cationic surfactants and polymers, high melting point fatty compounds, low melting point oils, silicone compounds, and mixtures thereof. Most of these conditioning agents are known to provide various conditioning benefits.

L'Oreal WO2012035065 discloses a cosmetic composition useful for, for example, hair treatment, comprising: volatile linear alkanes, plant oils, fatty ester(s) or alcohol(s), the ester is different from the plant oils. L'Oreal WO2012035065 describes that: volatile solvents used in hair care products may give rise to problems in terms of a greasy feel, lack of sheen, and stiff, hard hair, and it has been discovered that the combination of one or more volatile linear alkanes, one or more fatty alcohol(s) or ester(s) and one or more plant oils makes it possible to avoid the drawbacks mentioned above, and afford dry hair that is more supple and/or has a smoother feel and is shinier. L'Oreal WO2012035065 also discloses in example a cosmetic composition comprising (wt.%): 7% of sweet almond oil obtained by cold pressing, 15% of caprylic/capric acid triglycerides (60/40), 7% of unstabilized refined camellina oil (15/22/31/13 oleic/linoleic/linolenic/eicosenoic acid triglycerides), 15% of 2-octyldodecanol (15), and 55% of undecane/tridecane. However, this exemplified formula does not contain cationic surfactant, fatty alcohol, or aqueous carrier.
BEIERSDORF DE102005030864 discloses a cosmetic formulation for moisturizing skin, improving elasticity of skin, hair and nails, reducing cellulite and care of sensitive skin contains lotus blossom extract, and also discloses in example a water-in-oil emulsion containing 1.5% lotus blossom extract and water to 100%, 1.0% triglyceryl diisostearate, 2.0% hydrogenated coco glycerides, 0.5% octyldodecanol, 2.0% caprylic/capric triglycerides, together with other ingredients. However, this exemplified formula does not contain cationic surfactant or fatty alcohol.

However, there is still a need for providing improved hair manageability, for example, reduced volume on dry hair (especially at the bottom side of hair) and/or improved hair alignment on dry hair, while not deteriorating other benefits especially free flowing (i.e., reduced clumpy hair) on dry hair.

None of the existing art provides all of the advantages and benefits of the present invention.

### SUMMARY OF THE INVENTION

The present invention is directed to a hair conditioning composition comprising by weight: from about 0.1% to about 10% of a branched fatty alcohol having a melting point of below 25°C; from about 0.1% to about 15%, of triester; and
an aqueous carrier;
wherein the composition meets at least one of the following conditions:
   the composition further comprises from about 1.0% to about 10% of a cationic surfactant; and from about 2.5% to about 30% of a high melting point fatty compound having a melting point of 25°C or more; and
   the weight ratio of the branched fatty alcohol to the triester is from about 9:1 to about 1:5

The composition of the present invention provides improved hair manageability, for example, reduced volume on dry hair (especially at the bottom side of hair) and/or improved hair alignment on dry hair, while not deteriorating other benefits especially free flowing (i.e., reduced clumpy hair) on dry hair.

Hair manageability may be achieved by containing conditioning agents at higher levels. However, such compositions often compromise other benefits especially free flowing (i.e., reduced clumpy hair) on dry hair. The composition of the present invention may meet such contradictory needs which are often compromised when providing hair manageability.

### DETAILED DESCRIPTION OF THE INVENTION

While the specification concludes with claims particularly pointing out and distinctly claiming the invention, it is believed that the present invention will be better understood from the following description.

Herein, "comprising" means that other steps and other ingredients which do not affect the end result can be added. This term encompasses the terms "consisting of" and "consisting essentially of".

All percentages, parts and ratios are based upon the total weight of the compositions of the present invention, unless otherwise specified. All such weights as they pertain to listed ingredients are based on the active level and, therefore, do not include carriers or by-products that may be included in commercially available materials.

Herein, "mixtures" is meant to include a simple combination of materials and any compounds that may result from their combination.

The term "molecular weight" or "M.Wt." as used herein refers to the weight average molecular weight unless otherwise stated. The weight average molecular weight may be measured by gel permeation chromatography.

"QS" means sufficient quantity for 100%.

### HAIR CONDITIONING COMPOSITIONS

The hair conditioning composition of the present invention comprises: a hair conditioning composition comprising by weight:
from about 0.1% to about 10%, of a branched fatty alcohol having a melting point of below 25°C;
from about 0.1% to about 15% of triester; and
an aqueous carrier,
wherein the composition meets at least one of the following conditions:
   the composition further comprises from about 1.0% to about 10% of a cationic surfactant; and
   from about 2.5% to about 30% of a high melting point fatty compound having a melting point of 25°C or more; and
   the weight ratio of the branched fatty alcohol to the triester is from about 9:1 to about 1:5.

In the present invention, the weight ratio of the branched fatty alcohol to the triester is preferably from about 7:1 to about 1:4, more preferably from about 7:1 to about 1:3, still more preferably from about 7:1 to about 1:2, even more preferably from about 7:1 to 1:1. When the ratio of the branched fatty alcohol is too high, the composition may start to provide increased hair volume yet reduced hair manageability. When the ratio of the triester is too high, the composition may start to provide reduced free-flowing hair.

### BRANCHED FATTY ALCOHOL

The composition of the present invention comprises a branched fatty alcohol having a melting point below 25oC (excluding 25oC), preferably 20oC or below, more preferably 10oC or below, still more preferably 0oC or below. The branched fatty alcohol is included in the hair conditioning composition at a level of from about 0.1% to about 10%, preferably from about 0.3% to about 8%, more preferably from about 0.5% to about 6%. When the percentage of the branched fatty alcohol is too low, the composition may provide reduced hair free flow. When the percentage of the branched fatty alcohol is too high, the composition may provide reduced hair manageability and/or reduced hair free flow.

Branched fatty alcohols useful herein are those having a longer parent chain and a shorter branched chain, both chains being saturated hydrocarbons. The branched fatty alcohols preferably have from about 6 to about 40 carbon atoms, preferably from about10 to about 30 carbon atoms, more preferably from about 12 to about 24 carbon atoms in total. The longer parent chain is saturated hydrocarbon having preferably from about 4 to about 20 carbon atoms, and more preferably from about 6 to about 14 carbon atoms, still more preferably from about 6 to about 12 carbon atoms. The shorter branched chain is a saturated hydrocarbon having from about 2 to about 18 carbon atoms, and more preferably from about 4 to about 10 carbon atoms, still more preferably from about 6 to about 8 carbon atoms. The position of the branched chain can start from the second, third, fourth and fifth carbon of the parent chain, and preferably starts from the second carbon of the longer parent chain according to IUPAC rules.

Highly preferred branched fatty alcohol is 2-BEXYL-1-DECANOL, which has the following chemical structure:

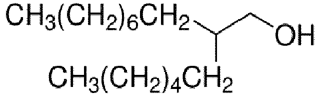

### TRIESTER / TRIGLYCERIDE

The composition of the present invention comprises a triester, preferably, triglyceride. The triester/triglyceride is included in the hair conditioning composition at a level from about 0.1% to about 15%, preferably from about 0.3% to about 12%, more preferably from about 0.5% to about 10%.

When the percentage of the triester is too low, the composition may provide reduced hair manageability. When the percentage of the triester is too high, the composition may provide reduced hair free flow.

Preferred are those having a melting point below 25°C, preferably 20°C or below, more preferably 10°C or below, still more preferably 0°C or below.

The triesters/triglycerides have three hydrocarbon groups coming from three hydrocarbon acids esterified respectively, preferably with glycerin, wherein each of the hydrocarbon groups has preferably from about 6 to about 40 carbon atoms, more preferably from about 6 to about 35 carbon atoms, still more preferably from about 6 to about 22 carbon atoms, even more preferably 6 to about 18 carbon atoms, further more preferably from about 6 to about 12 carbon atoms, even more preferably from about 8 to about 10 carbon atoms. The three hydrocarbon groups are independently, branched or straight, saturated or unsaturated.

Such triesters include, for example, glycerol triesters, i.e., triglycerides, having the following formula: 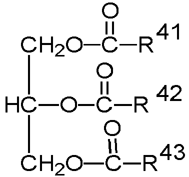 wherein R⁴¹, R⁴², and R⁴³, are explained above as three large head groups. Such glycerol triesters include, for example, trioctanoin (also known as glycerol trioctanoate or caprylic acid triglyceride), caprylic/capric triglyceride, and glyceryl tri (2-ethylhexanoate).

Such triesters also include, for example, trimethylol triesters having the following formula: 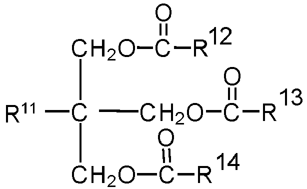 wherein R¹¹ is H, CH₃ or C₂H₅, and R¹², R¹³, and R¹⁴ are explained above as three large head groups.

Such triesters further include, for example, citrate triesters the following formula: 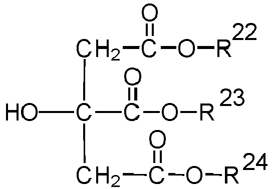 wherein R²², R²³, and R²⁴, are explained above as three large head groups.

In the present invention, it is preferred that the triester is a combination of: a triester having non-branched hydrocarbon groups; and a triester having branched hydrocarbon groups. The weight ratio of the non-branched triester to the branched triester is preferably from about 1:3 to about 3: 1. When the ratio of the non-branched triester becomes higher, the composition may start to provide reduced hair free flow. When the ratio of the branched triester becomes higher, the composition may start to provide increased hair volume, yet reduced hair manageability

In the non-branched triesters useful herein, the all three hydrocarbon groups are non-branched, each of the non-branched hydrocarbon groups can be saturated, monounsaturated or polyunsaturated, preferably saturated. Each of the non-branched hydrocarbon groups has preferably from about 6 to about 22 carbon atoms, more preferably 6 to about 18 carbon atoms, still more preferably from about 6 to about 12 carbon atoms, even more preferably from about 8 to about 10 carbon atoms. Highly preferred non-branched triester herein is non-branched triglyceride, and further preferred is caprylic/capric triglyceride.

In the branched triester useful herein, wherein the all three hydrocarbon groups are branched, each of the branched hydrocarbon groups can be saturated, monounsaturated or polyunsaturated, preferably saturated. Each of the branched hydrocarbon groups has preferably from about 6 to about 22 carbon atoms, more preferably 6 to about 18 carbon atoms, still more preferably from about 6 to about 12 carbon atoms, even more preferably from about 8 to about 10 carbon atoms.

Branched triester useful herein are those having a longer parent chain and a shorter branched chain, both chains being saturated hydrocarbons. Each of the longer parent chain is saturated hydrocarbon having preferably from about 4 to about 20 carbon atoms, and more preferably from about 6 to about 12 carbon atoms, still more preferably from about 6 to about 10 carbon atoms, even more preferably from about 6 to about 8 carbon atoms. The shorter branched chain is a saturated hydrocarbon having from about 1 to about 10 carbon atoms, and more preferably from about 2 to about 8 carbon atoms, still more preferably from about 2 to about 4 carbon atoms. The position of the branched chain can start from the second, third, fourth and fifth carbon of the parent chain, and preferably starts from the second carbon of the longer parent chain according to IUPAC rules.

Highly preferred branched triester herein is branched triglyceride, and further preferred is glyceryl tri (2-ethylhexanoate) having the following structure:

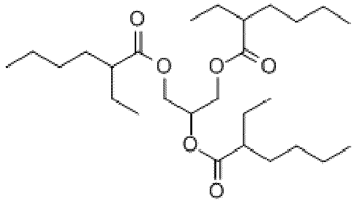

### CATIONIC SURFACTANT

The compositions of the present invention comprise a cationic surfactant. The cationic surfactant can be included in the composition at a level of from about 1.0%, preferably from about 1.5%, more preferably from about 2.0%, still more preferably from about 3.0%, and to about 25%, preferably to about 10%, more preferably to about 8.0%, still more preferably to about 6.0% by weight of the composition, in view of providing the benefits of the present invention.

Preferably, in the present invention, the surfactant is water-insoluble. In the present invention, "water-insoluble surfactants" means that the surfactants have a solubility in water at 25°C of preferably below 0.5g/100g (excluding 0.5g/100g) water, more preferably 0.3g/100g water or less.

Cationic surfactant useful herein can be one cationic surfactant or a mixture of two or more cationic surfactants. Preferably, the cationic surfactant is selected from: a mono-long alkyl quaternized ammonium salt; a combination of a mono-long alkyl quaternized ammonium salt and a di-long alkyl quaternized ammonium salt; a mono-long alkyl amine; a combination of a mono-long alkyl amine and a di-long alkyl quaternized ammonium salt; and a combination of a mono-long alkyl amine and a mono-long alkyl quaternized ammonium salt.

### Mono-long alkyl amine

Mono-long alkyl amine useful herein are those having one long alkyl chain of preferably from 12 to 30 carbon atoms, more preferably from 16 to 24 carbon atoms, still more preferably from 18 to 22 alkyl group. Mono-long alkyl amines useful herein also include mono-long alkyl amidoamines. Primary, secondary, and tertiary fatty amines are useful.

Particularly useful are tertiary amido amines having an alkyl group of from about 12 to about 22 carbons. Exemplary tertiary amido amines include: stearamidopropyldimethylamine, stearamidopropyldiethylamine, stearamidoethyldiethylamine, stearamidoethyldimethylamine, palmitamidopropyldimethylamine, palmitamidopropyldiethylamine, palmitamidoethyldiethylamine, palmitamidoethyldimethylamine, behenamidopropyldimethylamine, behenamidopropyldiethyl amine, behenamidoethyldiethyl amine, behenamidoethyldimethylamine, arachidamidopropyldimethylamine, arachidamidopropyldiethyl amine, arachidamidoethyldiethylamine, arachidamidoethyldimethylamine, diethylaminoethylstearamide. Useful amines in the present invention are disclosed in U.S. Patent 4,275,055, Nachtigal, et al.

These amines are used in combination with acids such as L-glutamic acid, lactic acid, hydrochloric acid, malic acid, succinic acid, acetic acid, fumaric acid, tartaric acid, citric acid, L-glutamic hydrochloride, maleic acid, and mixtures thereof; more preferably L-glutamic acid, lactic acid, citric acid, at a molar ratio of the amine to the acid of from about 1:0.3 to about 1:2, more preferably from about 1:0.4 to about 1:1.

### Mono-long alkyl quaternized ammonium salt

The mono-long alkyl quaternized ammonium salts useful herein are those having one long alkyl chain which has from 12 to 30 carbon atoms, preferably from 16 to 24 carbon atoms, more preferably C18-22 alkyl group. The remaining groups attached to nitrogen are independently selected from an alkyl group of from 1 to about 4 carbon atoms or an alkoxy, polyoxyalkylene, alkylamido, hydroxyalkyl, aryl or alkylaryl group having up to about 4 carbon atoms.

Mono-long alkyl quaternized ammonium salts useful herein are those having the formula (I): 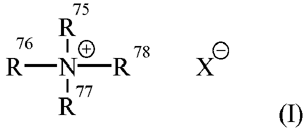 wherein one of R⁷⁵, R⁷⁶, R⁷⁷ and R⁷⁸ is selected from an alkyl group of from 12 to 30 carbon atoms or an aromatic, alkoxy, polyoxyalkylene, alkylamido, hydroxyalkyl, aryl or alkylaryl group having up to about 30 carbon atoms; the remainder of R⁷⁵, R⁷⁶, R⁷⁷ and R⁷⁸ are independently selected from an alkyl group of from 1 to about 4 carbon atoms or an alkoxy, polyoxyalkylene, alkylamido, hydroxyalkyl, aryl or alkylaryl group having up to about 4 carbon atoms; and X⁻ is a salt-forming anion such as those selected from halogen, (e.g. chloride, bromide), acetate, citrate, lactate, glycolate, phosphate, nitrate, sulfonate, sulfate, alkylsulfate, and alkyl sulfonate radicals. The alkyl groups can contain, in addition to carbon and hydrogen atoms, ether and/or ester linkages, and other groups such as amino groups. The longer chain alkyl groups, e.g., those of about 12 carbons, or higher, can be saturated or unsaturated. Preferably, one of R⁷⁵, R⁷⁶, R⁷⁷ and R⁷⁸ is selected from an alkyl group of from 12 to 30 carbon atoms, more preferably from 16 to 24 carbon atoms, still more preferably from 18 to 22 carbon atoms, even more preferably 22 carbon atoms; the remainder of R⁷⁵, R⁷⁶, R⁷⁷ and R⁷⁸ are independently selected from CH₃, C₂H₅, C₂H₄OH, and mixtures thereof; and X is selected from the group consisting of Cl, Br, CH₃OSO₃, C₂H₅OSO₃, and mixtures thereof.

Nonlimiting examples of such mono-long alkyl quaternized ammonium salt cationic surfactants include: behenyl trimethyl ammonium salt; stearyl trimethyl ammonium salt; cetyl trimethyl ammonium salt; and hydrogenated tallow alkyl trimethyl ammonium salt.

### Di-long alkyl quaternized ammonium salts

When used, di-long alkyl quaternized ammonium salts are preferably combined with a mono-long alkyl quaternized ammonium salt and/or mono-long alkyl amine salt, at the weight ratio of from 1:1 to 1:5, more preferably from 1:1.2 to 1:5, still more preferably from 1:1.5 to 1:4, in view of stability in rheology and conditioning benefits.

Di-long alkyl quaternized ammonium salts useful herein are those having two long alkyl chains of from 12 to 30 carbon atoms, more preferably from 16 to 24 carbon atoms, still more preferably from 18 to 22 carbon atoms. Such di-long alkyl quaternized ammonium salts useful herein are those having the formula (I): 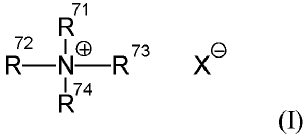 wherein two of R⁷¹, R⁷², R⁷³ and R⁷⁴ are selected from an aliphatic group of from 12 to 30 carbon atoms, preferably from 16 to 24 carbon atoms, more preferably from 18 to 22 carbon atoms or an aromatic, alkoxy, polyoxyalkylene, alkylamido, hydroxyalkyl, aryl or alkylaryl group having up to about 30 carbon atoms; the remainder of R⁷¹, R⁷², R⁷³ and R⁷⁴ are independently selected from an aliphatic group of from 1 to about 8 carbon atoms, preferably from 1 to 3 carbon atoms or an aromatic, alkoxy, polyoxyalkylene, alkylamido, hydroxyalkyl, aryl or alkylaryl group having up to about 8 carbon atoms; and X⁻ is a salt-forming anion selected from the group consisting of halides such as chloride and bromide, C1-C4 alkyl sulfate such as methosulfate and ethosulfate, and mixtures thereof. The aliphatic groups can contain, in addition to carbon and hydrogen atoms, ether linkages, and other groups such as amino groups. The longer chain aliphatic groups, e.g., those of about 16 carbons, or higher, can be saturated or unsaturated. Preferably, two of R⁷¹, R⁷², R⁷³ and R⁷⁴ are selected from an alkyl group of from 12 to 30 carbon atoms, preferably from 16 to 24 carbon atoms, more preferably from 18 to 22 carbon atoms; and the remainder of R⁷¹, R⁷², R⁷³ and R⁷⁴ are independently selected from CH₃, C₂H₅, C₂H₄OH, CH₂C₆H₅, and mixtures thereof.

Such preferred di-long alkyl cationic surfactants include, for example, dialkyl (14-18) dimethyl ammonium chloride, ditallow alkyl dimethyl ammonium chloride, dihydrogenated tallow alkyl dimethyl ammonium chloride, distearyl dimethyl ammonium chloride, and dicetyl dimethyl ammonium chloride.

### HIGH MELTING POINT FATTY COMPOUND

The composition of the present invention comprises a high melting point fatty compound. The high melting point fatty compound can be included in the composition at a level of from about 2.5%, preferably from about 3.0%, more preferably from about 4.0%, still more preferably from about 5.0%, and to about 30%, preferably to about 10%, more preferably to about 8.0% by weight of the composition, in view of providing the benefits of the present invention.

The high melting point fatty compound useful herein have a melting point of 25°C or higher, preferably 40°C or higher, more preferably 45°C or higher, still more preferably 50°C or higher, in view of stability of the emulsion especially the gel matrix. Preferably, such melting point is up to about 90°C, more preferably up to about 80°C, still more preferably up to about 70°C, even more preferably up to about 65°C, in view of easier manufacturing and easier emulsification. In the present invention, the high melting point fatty compound can be used as a single compound or as a blend or mixture of at least two high melting point fatty compounds. When used as such blend or mixture, the above melting point means the melting point of the blend or mixture.

The high melting point fatty compound useful herein is selected from the group consisting of fatty alcohols, fatty acids, fatty alcohol derivatives, fatty acid derivatives, and mixtures thereof. It is understood by the artisan that the compounds disclosed in this section of the specification can in some instances fall into more than one classification, e.g., some fatty alcohol derivatives can also be classified as fatty acid derivatives. However, a given classification is not intended to be a limitation on that particular compound, but is done so for convenience of classification and nomenclature. Further, it is understood by the artisan that, depending on the number and position of double bonds, and length and position of the branches, certain compounds having certain required carbon atoms may have a melting point of less than the above preferred in the present invention. Such compounds of low melting point are not intended to be included in this section. Nonlimiting examples of the high melting point compounds are found in International Cosmetic Ingredient Dictionary, Fifth Edition, 1993, and CTFA Cosmetic Ingredient Handbook, Second Edition, 1992.

Among a variety of high melting point fatty compounds, fatty alcohols are preferably used in the composition of the present invention. The fatty alcohols useful herein are those having from about 14 to about 30 carbon atoms, preferably from about 16 to about 22 carbon atoms. These fatty alcohols are saturated and can be straight or branched chain alcohols.

Preferred fatty alcohols include, for example, cetyl alcohol (having a melting point of about 56°C), stearyl alcohol (having a melting point of about 58-59°C), behenyl alcohol (having a melting point of about 71°C), and mixtures thereof. These compounds are known to have the above melting point. However, they often have lower melting points when supplied, since such supplied products are often mixtures of fatty alcohols having alkyl chain length distribution in which the main alkyl chain is cetyl, stearyl or behenyl group.

In the present invention, more preferred fatty alcohol is a mixture of cetyl alcohol and stearyl alcohol.

Generally, in the mixture, the weight ratio of cetyl alcohol to stearyl alcohol is preferably from about 1:9 to 9:1, more preferably from about 1:4 to about 4:1, still more preferably from about 1:2.3 to about 1.5:1.

When using higher level of total cationic surfactant and high melting point fatty compounds, the mixture has the weight ratio of cetyl alcohol to stearyl alcohol of preferably from about 1:1 to about 4:1, more preferably from about 1:1 to about 2:1, still more preferably from about 1.2:1 to about 2:1, in view of avoiding to get too thick for spreadability. It may also provide more conditioning on damaged part of the hair.

### AQUEOUS CARRIER

The composition of the present invention comprises an aqueous carrier. The level and species of the carrier are selected according to the compatibility with other components, and other desired characteristic of the product.

The carrier useful in the present invention includes water and water solutions of lower alkyl alcohols. The lower alkyl alcohols useful herein are monohydric alcohols having 1 to 6 carbons, more preferably ethanol and isopropanol.

Preferably, the aqueous carrier is substantially water. Deionized water is preferably used. Water from natural sources including mineral cations can also be used, depending on the desired characteristic of the product. Generally, the compositions of the present invention comprise from about 40% to about 99%, preferably from about 50% to about 95%, and more preferably from about 70% to about 90%, and more preferably from about 80% to about 90% aqueous carrier, preferably water.

### GEL MATRIX

Preferably, in the present invention, a gel matrix is formed by the cationic surfactant, the high melting point fatty compound, and an aqueous carrier. The gel matrix is suitable for providing various conditioning benefits, such as slippery feel during the application to wet hair and softness and moisturized feel on dry hair. Also, when combining this gel matrix with the above cyclic compound and the compound having at least three large head groups, the composition provides improved spreadability which is believed to provide more balanced deposition of hair conditioning agents both on hair tips and hair root and thus deliver further improved hair manageability.

Preferably, when the gel matrix is formed, the cationic surfactant and the high melting point fatty compound are contained at a level such that the weight ratio of the cationic surfactant to the high melting point fatty compound is in the range of, preferably from about 1:1 to about 1: 10, more preferably from about 1:1.5 to about 1:7, still more preferably from about 1:2 to about 1:6, in view of providing improved wet conditioning benefits.

Preferably, when the gel matrix is formed, the composition of the present invention is substantially free of anionic surfactants, in view of stability of the gel matrix. In the present invention, "the composition being substantially free of anionic surfactants" means that: the composition is free of anionic surfactants; or, if the composition contains anionic surfactants, the level of such anionic surfactants is very low. In the present invention, a total level of such anionic surfactants, if included, preferably 1% or less, more preferably 0.5% or less, still more preferably 0.1% or less by weight of the composition. Most preferably, the total level of such anionic surfactants is 0% by weight of the composition.

### SILICONE COMPOUND

The compositions of the present invention may further contain a silicone compound. It is believed that the silicone compound can provide smoothness and softness on dry hair. The silicone compounds herein can be used at levels by weight of the composition of preferably from about 0.1% to about 20%, more preferably from about 0.5% to about 10%, still more preferably from about 1% to about 8%.

Preferably, the silicone compounds have an average particle size of from about 1 micron to about 50 microns, in the composition.

The silicone compounds useful herein, as a single compound, as a blend or mixture of at least two silicone compounds, or as a blend or mixture of at least one silicone compound and at least one solvent, have a viscosity of preferably from about 1,000 to about 2,000,000mPa·s at 25°C.

The viscosity can be measured by means of a glass capillary viscometer as set forth in Dow Coming Corporate Test Method CTM0004, July 20, 1970. Suitable silicone fluids include polyalkyl siloxanes, polyaryl siloxanes, polyalkylaryl siloxanes, polyether siloxane copolymers, amino substituted silicones, quaternized silicones, and mixtures thereof. Other nonvolatile silicone compounds having conditioning properties can also be used.

Preferred polyalkyl siloxanes include, for example, polydimethylsiloxane, polydiethylsiloxane, and polymethylphenylsiloxane. Polydimethylsiloxane, which is also known as dimethicone, is especially preferred.

The above polyalkylsiloxanes are available, for example, as a mixture with silicone compounds having a lower viscosity. Such mixtures have a viscosity of preferably from about 1,000mPa·s to about 100,000mPa·s, more preferably from about 5,000mPa·s to about 50,000mPa ·s. Such mixtures preferably comprise: (i) a first silicone having a viscosity of from about 100,000mPa·s to about 30,000,000mPa·s at 25°C, preferably from about 100,000mPa·s to about 20,000,000mPa·s; and (ii) a second silicone having a viscosity of from about 5mPa·s to about 10,000mPa·s at 25°C, preferably from about 5mPa·s to about 5,000mPa·s. Such mixtures useful herein include, for example, a blend of dimethicone having a viscosity of 18,000,000mPa·s and dimethicone having a viscosity of 200mPa s available from GE Toshiba, and a blend of dimethicone having a viscosity of 18,000,000mPa·s and cyclopentasiloxane available from GE Toshiba.

The silicone compounds useful herein also include a silicone gum. The term "silicone gum", as used herein, means a polyorganosiloxane material having a viscosity at 25°C of greater than or equal to 1,000,000 centistokes. It is recognized that the silicone gums described herein can also have some overlap with the above-disclosed silicone compounds. This overlap is not intended as a limitation on any of these materials. The "silicone gums" will typically have a mass molecular weight in excess of about 200,000, generally between about 200,000 and about 1,000,000. Specific examples include polydimethylsiloxane, poly(dimethylsiloxane methylvinylsiloxane) copolymer, poly(dimethylsiloxane diphenylsiloxane methylvinylsiloxane) copolymer and mixtures thereof. The silicone gums are available, for example, as a mixture with silicone compounds having a lower viscosity. Such mixtures useful herein include, for example, Gum/Cyclomethicone blend available from Shin-Etsu.

Silicone compounds useful herein also include amino substituted materials. Preferred aminosilicones include, for example, those which conform to the general formula (I):

(R₁)ₐG₃₋ₐ-Si-(-OSiG₂)ₙ-(-OSi G_{b}(R₁)_{2-b})ₘ-O-SiG₃₋ₐ(R₁)ₐ

wherein G is hydrogen, phenyl, hydroxy, or C₁-C₈ alkyl, preferably methyl; a is 0 or an integer having a value from 1 to 3, preferably 1; b is 0, 1 or 2, preferably 1; n is a number from 0 to 1,999; m is an integer from 0 to 1,999; the sum of n and m is a number from 1 to 2,000; a and m are not both 0; R₁ is a monovalent radical conforming to the general formula CqH_{2q}L, wherein q is an integer having a value from 2 to 8 and L is selected from the following groups: -N(R₂)CH₂-CH₂-N(R₂)₂; -N(R₂)₂; -N(R₂)₃A ; -N(R₂)CH₂-CH₂-NR₂H₂A ; wherein R₂ is hydrogen, phenyl, benzyl, or a saturated hydrocarbon radical, preferably an alkyl radical from about C₁ to about C₂₀; A is a halide ion.

Highly preferred amino silicones are those corresponding to formula (I) wherein m=0, a=1, q=3, G=methyl, n is preferably from about 1500 to about 1700, more preferably about 1600; and L is -N(CH₃)₂ or -NH₂, more preferably -NH₂. Another highly preferred amino silicones are those corresponding to formula (I) wherein m=0, a=1, q=3, G=methyl, n is preferably from about 400 to about 600, more preferably about 500; and L is -N(CH₃)₂ or -NH₂, more preferably -NH₂. Such highly preferred amino silicones can be called as terminal aminosilicones, as one or both ends of the silicone chain are terminated by nitrogen containing group.

The above aminosilicones, when incorporated into the composition, can be mixed with solvent having a lower viscosity. Such solvents include, for example, polar or non-polar, volatile or non-volatile oils. Such oils include, for example, silicone oils, hydrocarbons, and esters. Among such a variety of solvents, preferred are those selected from the group consisting of non-polar, volatile hydrocarbons, volatile cyclic silicones, non-volatile linear silicones, and mixtures thereof. The non-volatile linear silicones useful herein are those having a viscosity of from about 1 to about 20,000 centistokes, preferably from about 20 to about 10,000 centistokes at 25°C. Among the preferred solvents, highly preferred are non-polar, volatile hydrocarbons, especially non-polar, volatile isoparaffins, in view of reducing the viscosity of the aminosilicones and providing improved hair conditioning benefits such as reduced friction on dry hair. Such mixtures have a viscosity of preferably from about 1,000mPa·s to about 100,000mPa ·s, more preferably from about 5,000mPa·s to about 50,000mPa·s.

Other suitable alkylamino substituted silicone compounds include those having alkylamino substitutions as pendant groups of a silicone backbone. Highly preferred are those known as "amodimethicone". Commercially available amodimethicones useful herein include, for example, BY16-872 available from Dow Corning.

The silicone compounds may further be incorporated in the present composition in the form of an emulsion, wherein the emulsion is made my mechanical mixing, or in the stage of synthesis through emulsion polymerization, with or without the aid of a surfactant selected from anionic surfactants, nonionic surfactants, cationic surfactants, and mixtures thereof.

### Silicone Polymer Containing Quaternary Groups

Silicone compounds useful herein include, for example, a Silicone Polymer Containing Quaternary Groups comprising terminal ester groups, having a viscosity up to 100,000 mPa s and a D block length of greater than 200 D units. Without being bound by theory, this low viscosity silicone polymer provides improved conditioning benefits such as smooth feel, reduced friction, and prevention of hair damage, while eliminating the need for a silicone blend.

Structurally, the silicone polymer is a polyorganosiloxane compound comprising one or more quaternary ammonium groups, at least one silicone block comprising greater than 200 siloxane units, at least one polyalkylene oxide structural unit, and at least one terminal ester group. In one or more embodiments, the silicone block may comprise between 300 to 500 siloxane units.

The silicone polymer is present in an amount of from about 0.05% to about 15%, preferably from about 0.1% to about 10%, more preferably from about 0.15% to about 5%, and even more preferably from about 0.2% to about 4% by weight of the composition.

In a preferred embodiment, the polyorganosiloxane compounds have the general formulas (Ia) and (Ib):

M-Y-L-(N⁺R₂-T-N⁺R₂)-Y-]ₘ-L-(NR²-A-E-A'-NR²)-Y-]ₖ-M (Ia)

M-Y-[-(N⁺R₂-T-N⁺R₂)-Y-]ₘ-[-(N⁺R²₂-A-E-A'-N⁺R²₂)-Y-]ₖ-M (Ib)

wherein:
m is > 0, preferred 0.01 to 100, more preferred 0.1 to 100, even more preferred 1 to 100, specifically 1 to 50, more specifically 1 to 20, even more specifically 1 to 10,
k is 0 or an average value of from >0 to 50, or preferably from 1 to 20, or even more preferably from 1 to 10,
M represents a terminal group, comprising terminal ester groups selected from

   -OC(O)-Z

   -OS(O) ₂-Z

   -OS(O₂)O-Z

   -OP(O)(O-Z)OH

   -OP(O)(O-Z) ₂
wherein Z is selected from monovalent organic residues having up to 40 carbon atoms, optionally comprising one or more hetero atoms.
A and A' each are independently from each other selected from a single bond or a divalent organic group having up to 10 carbon atoms and one or more hetero atoms, and
E is a polyalkylene oxide group of the general formula:

   -[CH₂CH₂O]_{q}-[CH₂CH(CH₃)O]ᵣ-[CH₂CH(C₂H₅)O]ₛ-
wherein q=0 to 200, r=0 to 200, s=0 to 200, and q+r+s = 1 to 600.
R² is selected from hydrogen or R,
R is selected from monovalent organic groups having up to 22 carbon atoms and optionally one or more heteroatoms, and wherein the free valencies at the nitrogen atoms are bound to carbon atoms, Y is a group of the formula:

   -K-S-K- and -A-E-A'-or-A'-E-A-, 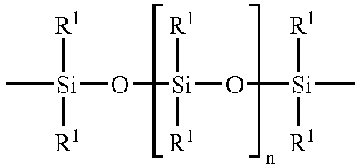
with S=
wherein R1 = C₁-C₂₂-alkyl, C₁-C₂₂-fluoralkyl or aryl; n=200 to 1000, and these can be identical or different if several S Groups are present in the polyorganosiloxane compound.
K is a bivalent or trivalent straight chain, cyclic and/or branched C₂-C₄₀ hydrocarbon residue which is optionally interrupted by-O-,-NH-, trivalent N, -NR¹-,-C(O)-, -C(S)-, and optionally substituted with-OH, wherein R¹ is defined as above,
T is selected from a divalent organic group having up to 20 carbon atoms and one or more hetero atoms.

The residues K may be identical or different from each other. In the -K-S-K- moiety, the residue K is bound to the silicon atom of the residue S via a C-Si-bond.

Due to the possible presence of amine groups (-(NR²-A-E-A'-NR²)-) in the polyorganosiloxane compounds, they may have protonated ammonium groups, resulting from the protonation of such amine groups with organic or inorganic acids. Such compounds are sometimes referred to as acid addition salts of the polyorganosiloxane compounds.

In a preferred embodiment the molar ratio of the quaternary ammonium groups b) and the terminal ester groups c) is less than 100:20, even more preferred is less than 100:30 and is most preferred less than 100:50. The ratio can be determined by ¹³C-NMR.

In a further embodiment, the polyorganosiloxane composition may comprise:
A) at least one polyorganosiloxane compound, comprising a) at least one polyorganosiloxane group, b) at least one quaternary ammonium group, c) at least one terminal ester group, and d) at least one polyalkylene oxide group (as defined before),
B) at least one polyorganosiloxane compound, comprising at least one terminal ester group, different from compound A).

In the definition of component A) it can be referred to the description of the polyorganosiloxane compounds of the invention. The polyorganosiloxane compound B) differs from the polyorganosiloxane compound A) preferably in that it does not comprise quaternary ammonium groups. Preferred polyorganosiloxane compounds B) result from the reaction of monofunctional organic acids, in particular carboxylic acids, and polyorganosiloxane containing bisepoxides.

In the polyorganosiloxane compositions the weight ratio of compound A) to compound B) is preferably less than 90:10. Or in other words, the content of component B) is at least 10 weight percent. In a further preferred embodiment of the polyorganosiloxane compositions in compound A) the molar ratio of the quaternary ammonium groups b) and the terminal ester groups c) is less than 100:10, even more preferred is less than 100:15 and is most preferred less than 100:20.

The silicone polymer has a viscosity at 20°C and a shear rate of 0.1s⁻¹ (plate-plate system, plate diameter 40mm, gap width 0.5mm) of less than 100,000 mPa•s (100 Pa•s). In further embodiments, the viscosities of the neat silicone polymers may range from 500 to 100,000 mPa•s, or preferably from 500 to 70,000 mPa•s, or more preferably from 500 to 50,000 mPa•s, or even more preferably from 500 to 20,000 mPa•s. In further embodiments, the viscosities of the neat polymers may range from 500 to 10,000 mPa•s, or preferably 500 to 5000 mPa•s determined at 20 °C and a shear rate of 0.1 s⁻¹.

In addition to the above listed silicone polymers, the following preferred compositions are provided below. For example, in the polyalkylene oxide group E of the general formula:

-[CH₂CH₂O]_{q}-[CH₂CH(CH₃)O]ᵣ-[CH₂CH(C₂H₅)O]ₛ-

wherein the q, r, and s indices may be defined as follows:
q = 0 to 200, or preferably from 0 to 100, or more preferably from 0 to 50, or even more preferably from 0 to 20,
r = 0 to 200, or preferably from 0 to 100, or more preferably from 0 to 50, or even more preferably from 0 to 20,
s = 0 to 200, or preferably from 0 to 100, or more preferably from 0 to 50, or even more preferably from 0 to 20, and
q+r+s = 1 to 600, or preferably from 1 to 100, or more preferably from 1 to 50, or even more preferably from 1 to 40.

For polyorganosiloxane structural units with the general formula S: 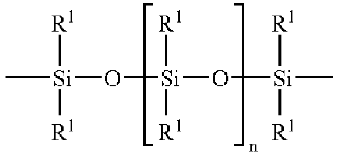 R¹=C₁-C₂₂-alkyl, C₁-C₂₂-fluoralkyl or aryl; n= from 200 to 1000, or preferably from 300 to 500, K (in the group -K-S-K-) is preferably a bivalent or trivalent straight chain, cyclical or branched C₂-C₂₀ hydrocarbon residue which is optionally interrupted by-O-,-NH-, trivalent N,-NR¹-,-C(O)-,-C(S)-, and optionally substituted with-OH.

In specific embodiments, R¹ is C₁-C₁₈ alkyl, C₁-C₁₈ fluoroalkyl and aryl. Furthermore, R¹ is preferably C₁-C₁₈ alkyl, C₁-C₆ fluoroalkyl and aryl. Furthermore, R¹ is more preferably C₁-C₆ alkyl, C₁-C₆ fluoroalkyl, even more preferably C₁-C₄ fluoroalkyl, and phenyl. Most preferably, R¹ is methyl, ethyl, trifluoropropyl and phenyl.

As used herein, the term "C₁-C₂₂ alkyl" means that the aliphatic hydrocarbon groups possess from 1 to 22 carbon atoms which can be straight chain or branched. Methyl, ethyl, propyl, n-butyl, pentyl, hexyl, heptyl, nonyl, decyl, undecyl, isopropyl, neopentyl and 1,2,3-trimethyl hexyl moieties serve as examples.

Further as used herein, the term "C₁-C₂₂ fluoroalkyl" means aliphatic hydrocarbon compounds with 1 to 22 carbon atoms which can be straight chain or branched and are substituted with at least one fluorine atom. Monofluormethyl, monofluoroethyl, 1,1,1-trifluorethyl, perfluoroethyl, 1,1,1-trifluoropropyl, 1,2,2-trifluorobutyl are suitable examples.

Moreover, the term "aryl" means unsubstituted or phenyl substituted once or several times with OH, F, Cl, CF₃, C₁-C₆ alkyl, C₁-C₆ alkoxy, C₃-C₇ cycloalkyl, C₂-C₆ alkenyl or phenyl. Aryl may also mean naphthyl.

For the embodiments of the polyorganosiloxanes, the positive charges resulting from the ammonium group(s), are neutralized with inorganic anions such as chloride, bromide, hydrogen sulfate, sulfate, or organic anions, like carboxylates deriving from C₁-C₃₀ carboxylic acids, for example acetate, propionate, octanoate, especially from C₁₀-C₁₈ carboxylic acids, for example decanoate, dodecanoate, tetradecanoate, hexadecanoate, octadecanoate and oleate, alkylpolyethercarboxylate, alkyl sulphonate, aryl sulphonate, alkylaryl sulphonate, alkylsulphate, alkylpolyethersulphate, phosphates derived from phosphoric acid mono alkyl/aryl ester and phosphoric acid dialkyl/aryl ester. The properties of the polyorganosiloxane compounds can be, *inter alia,* modified based upon the selection of acids used.

The quaternary ammonium groups are usually generated by reacting the di-tertiary amines with an alkylating agents, selected from in particular di-epoxides (sometimes referred to also as bis-epoxides) in the presence of mono carboxylic acids and difunctional dihalogen alkyl compounds.

In a preferred embodiment the polyorganosiloxane compounds are of the general formulas (Ia) and (Ib):

M-Y-L-(N⁺R₂-T-N⁺R₂)-Y-]ₘ-L-(NR²-A-E-A'-NR²)-Y-]ₖ-M (Ia)

M-Y-[-(N⁺R₂-T-N⁺R₂)-Y-]ₘ-[-(N⁺R²₂-A-E-A'-N⁺R²₂)-Y-]ₖ-M (Ib)

wherein each group is as defined above; however, the repeating units are in a statistical arrangement (i.e., not a block-wise arrangement).

In a further preferred embodiment the polyorganosiloxane compounds may be also of the general formulas (IIa) or (IIb):

M-Y-[-N⁺R₂-Y-]ₘ-[-(NR²-A-E-A'-NR²)-Y-]ₖ-M (IIa)

M-Y-L-N⁺R₂-Y-]ₘ-(N⁺R²₂-A-E-A'-N⁺R²₂)-Y-]ₖ-M (IIb)

wherein each group is as defined above. Also in such formula the repeating units are usually in a statistical arrangement (i.e. not a block-wise arrangement).
wherein, as defined above, M is

-OC(O)-Z,

-OS(O)₂-Z

-OS(O₂)O-Z

-OP(O)(O-Z)OH

-OP(O)(O-Z)₂

Z is a straight chain, cyclic or branched saturated or unsaturated C₁-C₂₀, or preferably C₂ to C₁₈, or even more preferably a hydrocarbon radical, which can be interrupted by one or more -O-, or -C(O)- and substituted with -OH. In a specific embodiment, M is -OC(O)-Z resulting from normal carboxylic acids in particular with more than 10 carbon atoms like for example dodecanoic acid.

In a further embodiment, the molar ratio of the polyorganosiloxane-containing repeating group -K-S-K-and the polyalkylene repeating group -A-E-A'- or -A'-E-A- is between 100:1 and 1:100, or preferably between 20:1 and 1:20, or more preferably between 10:1 and 1:10.

In the group -(N⁺R₂-T-N⁺R₂)-, R may represent a monovalent straight chain, cyclic or branched C₁-C₂₀ hydrocarbon radical, which can be interrupted by one or more -O- , - C(O)- and can be substituted by-OH, T may represent a divalent straight-chain, cyclic, or branched C₁-C₂₀ hydrocarbon radical, which can be interrupted by -O- , -C(O)- and can be substituted by hydroxyl.

The above described polyorganosiloxane compounds comprising quaternary ammonium functions and ester functions may also contain: 1) individual molecules which contain quaternary ammonium functions and no ester functions; 2) molecules which contain quaternary ammonium functions and ester functions; and 3) molecules which contain ester functions and no quaternary ammonium functions. While not limited to structure, the above described polyorganosiloxane compounds comprising quaternary ammonium functions and ester functions are to be understood as mixtures of molecules comprising a certain averaged amount and ratio of both moieties.

Various monofunctional organic acids may be utilized to yield the esters. Exemplary embodiments include C₁-C₃₀ carboxylic acids, for example C₂, C₃, C₈ acids, C₁₀-C₁₈ carboxylic acids, for example C₁₂, C₁₄, C₁₆ acids, saturated, unsaturated and hydroxyl functionalized C₁₈ acids, alkylpolyethercarboxylic acids, alkylsulphonic acids, arylsulphonic acids, alkylarylsulphonic acids, alkylsulphuric acids, alkylpolyethersulphuric acids, phosphoric acid mono alkyl/aryl esters and phosphoric acid dialkyl/aryl esters.

### ADDITIONAL COMPONENTS

The composition of the present invention may include other additional components, which may be selected by the artisan according to the desired characteristics of the final product and which are suitable for rendering the composition more cosmetically or aesthetically acceptable or to provide them with additional usage benefits. Such other additional components generally are used individually at levels of from about 0.001% to about 10%, preferably up to about 5% by weight of the composition.

A wide variety of other additional components can be formulated into the present compositions. These include: other conditioning agents such as hydrolysed collagen with tradename Peptein 2000 available from Hormel, vitamin E with tradename Emix-d available from Eisai, panthenol available from Roche, panthenyl ethyl ether available from Roche, hydrolysed keratin, proteins, plant extracts, and nutrients; preservatives such as sodium benzoate, phenoxyethanol, benzyl alcohol, methyl paraben, propyl paraben and imidazolidinyl urea; pH adjusting agents, such as citric acid, sodium citrate, succinic acid, phosphoric acid, sodium hydroxide, sodium carbonate; coloring agents, such as any of the FD&C or D&C dyes; perfumes; ultraviolet and infrared screening and absorbing agents such as benzophenones; and antidandruff agents such as zinc pyrithione; non-ionic surfactant such as mono-9-octadecanoate poly(oxy-1,2-ethanediyl) supplied as, for example, Tween 20; and buffer such as aminomethyl propanol.

### PRODUCT FORMS

The compositions of the present invention can be in the form of rinse-off products or leave-on products, and can be formulated in a wide variety of product forms, including but not limited to creams, gels, emulsions, mousses and sprays. The composition of the present invention is especially suitable for hair conditioners especially leave-on, leave-in, and/or no-rinse hair conditioners. Leave-on and leave-in hair conditioners are generally used on dry, semi-wet, and/or wet hair without rinsing out the conditioner. By no-rinse hair conditioners, what is meant herein is a hair conditioner used on semi-wet to wet hair after shampooing, without rinsing out the conditioner, preferably inside of bathroom.

### EXAMPLES

The following examples further describe and demonstrate embodiments within the scope of the present invention. The examples are given solely for the purpose of illustration and are not to be construed as limitations of the present invention, as many variations thereof are possible without departing from the spirit and scope of the invention. Where applicable, ingredients are identified by chemical or CTFA name, or otherwise defined below.

### Compositions (wt%)

| Components | Ex. 1 | Ex. 2 | Ex. 3 | Ex. 4 | CEx. i | CEx. ii | CEx. iii |
|---|---|---|---|---|---|---|---|
| Behenyl trimethylammonium methosulfate | 2.97 | 2.97 | 2.97 | 2.97 | 2.97 | 2.97 | 2.97 |
| Cetyl alcohol | 1.18 | 1.18 | 1.18 | 1.18 | 1.18 | 1.18 | 1.18 |
| Stearyl alcohol | 2.93 | 2.93 | 2.93 | 2.93 | 2.93 | 2.93 | 2.93 |
| Branched fatty alcohol-1 (2-Hexyl-1-decanol) | 4 | 4 | 0.61 | 2.97 | - | - | 4 |
| Non-branched triglyceride-1 (Caprylic/Capric triglyceride) | - | 0.9 | 4.87 | 0.76 | - | - | - |
| Branched triglyceride-1 (Glyceryl tri(2-ethylhenanoate)) | 1.8 | 1.8 | 1.22 | 2.97 | - | 1.8 | - |
| Silicone compound * 1 | 2.5 | 2.5 | 2.5 | 2.5 | 2.5 | 2.5 | 2.5 |
| Laureth-7 | - | 0.31 | 0.31 | 0.31 | - | - | - |
| Preservatives | 0.5 | 0.5 | 0.5 | 0.5 | 0.5 | 0.5 | 0.5 |
| Perfume | 0.5 | 0.5 | 0.5 | 0.5 | 0.5 | 0.5 | 0.5 |
| Deionized Water | q.s. to 100% of the composition | | | | | | |
| Preparation of hair switch | NR | NR | NR | NR | NR | NR | NR |
| Weight ratio of Branched fatty alcohol to Triglycerides | 2.2:1 | 1.5:1 | 1:10 | 1:1.25 | - | - | - |
| Volume | SS | SS | SS | S | C | E | C- |
| Free flow | - SS | SS | C+ | S | C | E | D |

### Compositions (wt%)

| Components | Ex. 5 | Ex. 6 | Ex. 7 | Ex. 8 |
|---|---|---|---|---|
| Behenyl trimethylammonium methosulfate | 2.97 | 2.97 | 2.97 | 2.97 |
| Cetyl alcohol | 1.18 | 1.18 | 1.18 | 1.18 |
| Stearyl alcohol | 2.93 | 2.93 | 2.93 | 2.93 |
| Branched fatty alcohol-1 (2-Hexyl-1-decanol) | 4 | 4 | 4 | 4 |
| Non-branched triglyceride-1 (Caprylic/Capric triglyceride) | 0.9 | 0.9 | 0.9 | 0.9 |
| Non-branched triglyceride-2 | - | - | - | - |
| Branched triglyceride-1 (Glyceryl tri(2-ethylhenanoate)) | 1.8 | 1.8 | 1.8 | 1.8 |
| Silicone compound *1 | - | - | - | - |
| Silicone compound *2 | 2.5 | - | - | - |
| Silicone compound *3 | - | 2.5 | - | - |
| Silicone compound *4 | - | - | 2.5 | - |
| Silicone compound *5 | - | - | - | 2.5 |
| Laureth-7 | 0.31 | 0.31 | 0.31 | 0.31 |
| Preservatives | 0.5 | 0.5 | 0.5 | 0.5 |
| Perfume | 0.5 | 0.5 | 0.5 | 0.5 |
| Deionized Water | q.s. to 100% of the composition | | | |
| Preparation of hair switch | NR | NR | NR | NR |
| Weight ratio of Branched fatty alcohol to Triglycerides | 1.5:1 | 1.5:1 | 1.5:1 | 1.5:1 |
| Volume | SS | SS | SS | SS |
| Free flow | SS | S | SS | SS |

### Definitions of Components

*1 Silicone compound-1: Available from Momentive having the following formula:

M-Y-[-(N⁺R₂-T-N⁺R₂)-Y-]ₘ-[-(N⁺R²₂-A-E-A'-N⁺R²₂)-Y-]ₖ-M

wherein

| | |
|---|---|
| M | lauric ester |
| Y | K-S-K |
| K | CH₂-CHOH-CH₂-O-C₃H₆ |
| S | PDMS block with 368 siloxane units |
| R, R² | Methyl |
| T | C₆H₁₂ |
| A | CH₂-COO- |
| A' | CO-CH₂ |
| E | Ethylene oxide (CH₂-CH₂-O) with average degree of ethoxylation of 2 |
| Ratio of silicone blocks : alkylene oxide blocks | 1:1 |
| Total Viscosity | 4700 mPa•s |

*2 Silicone compound-2: Available from Momentive having a viscosity 10,000mPa·s, and having following formula (I):

(R₁)ₐG₃₋ₐ-Si-(-OSiG₂)ₙ-(-OSiG_{b}(R₁)_{2-b})ₘ-O-SiG₃₋ₐ(R₁)ₐ (I)

wherein G is methyl; a is an integer of 1; b is 0, 1 or 2, preferably 1; n is a number from 400 to about 600; m is an integer of 0; Ri is a monovalent radical conforming to the general formula CqH_{2q}L, wherein q is an integer of 3 and L is -NH₂
*3 Silicone compound-3: 15%/85% mixture of dimethicone having a viscosity of 18,000,000 mm²·s⁻¹ and dimethicone having a viscosity of 200 mm²·s⁻¹ at 25°C
*4 Silicone compound-4: XIAMETER^{™} PMX-1503 Fluid, which is a blend of ultra-high viscosity dimethiconol in a low viscosity dimethicone fluid and has a viscosity of 1500cSt
*5 Silicone compound-5: Dimethicone having a viscosity of 10,000cSt

### Method of Preparation

The above hair care compositions of "Ex. 1" through "Ex. 8" of the present invention and hair care compositions of "CEx. i" through "CEx. iii" as comparative examples can be prepared by any conventional method well known in the art.

### Properties and Conditioning benefits

For some of the above compositions, properties and conditioning benefits are evaluated by the following methods. Results of the evaluation are also shown above in tables.

The embodiments disclosed and represented by "Ex. 1" through "Ex. 8" are hair conditioning compositions of the present invention which are particularly useful for the use on semi-wet to wet hair after shampooing, without rinsing out the conditioning composition. Such embodiments have many advantages. For example, they provide improved hair manageability such as reduced hair volume. Such advantages can be understood by the comparison between the example of the present invention "Ex. 1" through "Ex. 8" and the comparative examples "CEx.i and CEx.iii".

### Hair volume

### (i) Preparation of hair switch

For the volume measurement, 15 gram hair switch with a length of 10inch are used. The hair switches are prepared by following steps:
(1) The hair switches are bleached and combed in the same way. Then, applying 1.5cc of non-conditioning shampoo per one hair switch, lathering, rinsing and drying the hair switches;
(2) Applying a non-conditioning shampoo at a level of 1.5cc per one hair switch and lathering the hair switch; and rinsing the hair switch; and
(3) Applying conditioner at a level of 1.5cc per one hair switch and treating the hair switch; and
(4) not-rinsing the hair switch (described as "NR") or rinsing the hair switch (described as "R"); and
(5) Then drying the hair switch at 50°C and in low humidity (30%) environment for 1.5h;
(6) Then placing the hair switches in a high humidity (75%) environment, for 1h at 28 °C, in which the hair switches tend to expand and increase the volume.

Hair switches are ready for volume measurements.

### (ii) Volume measurements

Volume are measured by a method described in Canadian patent application publication No. CA2567712 A1. In more detail, the volume (mm3) is measured by conducting 3D reading by the laser scanning device.

### (iii) Evaluation

The above measurement in the step (ii) are conducted on at least 3 different hair switches prepared by the step (i) per one conditioner, and then calculate an average of the volume. The averaged hair volume is evaluated as follows.
SS: Above 55% (excluding 55%) decrease of volume, compared to Control.
S: Above 30% (excluding 30%) to 55% decrease of volume, compared to Control.
A: Above 15% (excluding 15%) to 30% decrease of volume, compared to Control.
B: Above 5% (excluding 5%) to 15% decrease of volume, compared to Control.
C+: Up to 5% (including 5%) decrease of volume, compared to Control.
C: Control.
C-: Up to 5% (including 5%) increase of volume, compared to Control.
D: Above 5% (excluding 5%) to 15% increase of volume, compared to Control.
E: Above 15% (excluding 15%) increase of volume, compared to Control.

### Free flow - Pendulum

### (i) Preparation of hair switch

For the pendulum measurement, 20-gram hair switch with a length of 10 inch are used. The hair switches are prepared by following steps:
(1) The hair switches are bleached and combed in the same way. Then, applying 1.0 cc of non-conditioning shampoo per one hair switch, lathering, rinsing and drying the hair switches;
(2) Applying a non-conditioning shampoo at a level of 1.0 cc per one hair switch and lathering the hair switch; and rinsing the hair switch;
(3) Applying conditioner (or treatment?) at a level of 2.0 cc per one hair switch for treatment and 4.0 cc per hair switch for conditioner and treating the hair switch; and
(4) not-rinsing the hair switch (described as "NR") or rinsing the hair switch (described as "R"); and
(5) Then drying the hair switch at 23°C and in low humidity (45%) environment overnight;

Hair switches are ready for pendulum measurements.

### (ii) Pendulum measurements

Free flow is measured by a pendulum measurement which detects degree of swinging of hair. More swinging means more free flowing. Treated hair is placed and a sensor to measure a degree of swinging is also placed. Same degree of starting force is given to the treated hair so that the treated hair starts swinging. The degree of swinging is measured during swinging.

### (iii) Evaluation

The above measurement in the step (ii) are conducted on at least 3 different hair switches prepared by the step (i) per one conditioner, and then calculate an average. The averaged free flowing is evaluated as follows.
SS Above 45% (excluding 45%) increase of free flow, compared to Control.
S: Above 30% (excluding 30%) to 45% increase of free flow, compared to Control.
A: Above 15% (excluding 15%) to 30% increase of free flow, compared to Control.
B: Above 5% (excluding 5%) to 15% increase of free flow, compared to Control.
C+: Up to 5% (including 5%) increase of free flow, compared to Control.
C: Control.
C-: Up to 5% (including 5%) decrease of free flow, compared to Control.
D: Above 5% (excluding 5%) to 15% decrease of free flow, compared to Control.
E: Above 15% (excluding 15%) decrease of free flow, compared to Control.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

Every document cited herein, including any cross referenced or related patent or application and any patent application or patent to which this application claims priority or benefit thereof, is hereby incorporated herein by reference in its entirety unless expressly excluded or otherwise limited. The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a document incorporated by reference, the meaning or definition assigned to that term in this document shall govern.

While particular embodiments of the present invention have been illustrated and described, it would be obvious to those skilled in the art that various other changes and modifications can be made without departing from the spirit and scope of the invention. It is therefore intended to cover in the appended claims all such changes and modifications that are within the scope of this invention.

### CLAIMS of EUROPEAN PATENT APPLICATION No. 20 751 043.9

1. A hair conditioning composition comprising by weight:
   from about 0.1% to about 10% of a branched fatty alcohol having a melting point of below 25°C;
   from about 0.1% to about 15%, of triester; and
   an aqueous carrier;
   wherein the composition meets at least one of the following conditions:
      the composition further comprises from about 1.0% to about 10% of a cationic surfactant; and
      from about 2.5% to about 30% of a high melting point fatty compound having a melting point of 25°C or more; and
      the weight ratio of the branched fatty alcohol to the triester is from about 9:1 to about 1:5.
2. The hair conditioning composition of Claim 1, wherein the weight ratio of the branched fatty alcohol to the triester is from about 7:1 to about 1:4, preferably from about 7:1 to about 1:3, more preferably from about 7:1 to about 1:2, still more preferably from about 7:1 to about 1:1.
3. The hair conditioning composition of any of the preceding claims, wherein the branched fatty alcohol has a melting point of below 20°C, preferably below 10°C.
4. The hair conditioning composition of any of the preceding claims, wherein the branched fatty alcohol has from about 12 to about 24 carbon atoms, preferably the branched fatty alcohol is 2-hexyl-1-decanol.
5. The hair conditioning composition of any of the preceding claims, wherein the triester are those having a melting point of 20oC or below, preferably 10oC or below.
6. The hair conditioning composition of any of the preceding claims, wherein the triester has three hydrocarbon groups derived from three hydrocarbon acids, and each of the three hydrocarbon groups has from about 6 to about 12 carbon atoms, preferably 8 to 10 carbon atoms.
7. The hair conditioning composition of any of the preceding claims, wherein the triester is a combination of: a triester having non-branched hydrocarbon groups; and a triester having branched hydrocarbon group.
8. The hair conditioning composition of any of the preceding claims, wherein the weight ratio of non-branched triester to branched triester is from about 1:3 to about 3: 1.
9. The hair conditioning composition of any of the preceding claims, wherein the non-branched triester is caprylic/capric triglyceride.
10. The hair conditioning composition of any of the preceding claims, wherein the branched triester is glyceryl tri (2-ethylhexanoate).

## Claims

1. A hair conditioning composition comprising by weight:
from 0.1% to 10% of a branched fatty alcohol having a melting point of below 25°C;
from 0.1% to 15%, of a triester; and
an aqueous carrier;
wherein the composition meets at least one of the following conditions:
the composition further comprises from 1.0% to 10% of a cationic surfactant; and from 2.5% to 30% of a high melting point fatty compound having a melting point of 25°C or more; and
the weight ratio of the branched fatty alcohol to the triester is from 9:1 to 1:5.

2. The hair conditioning composition of Claim 1, wherein the weight ratio of the branched fatty alcohol to the triester is from 7:1 to 1:4, preferably from 7:1 to 1:3, more preferably from 7:1 to 1:2, still more preferably from 7:1 to 1:1.

3. The hair conditioning composition of any of the preceding claims, wherein the branched fatty alcohol has a melting point of below 20°C, preferably below 10°C.

4. The hair conditioning composition of any of the preceding claims, wherein the branched fatty alcohol has from 12 to 24 carbon atoms, preferably the branched fatty alcohol is 2-hexyl-1-decanol.

5. The hair conditioning composition of any of the preceding claims, wherein the triester are those having a melting point of 20°C or below, preferably 10°C or below.

6. The hair conditioning composition of any of the preceding claims, wherein the triester has three hydrocarbon groups derived from three hydrocarbon acids, and each of the three hydrocarbon groups has from 6 to 12 carbon atoms, preferably 8 to 10 carbon atoms.

7. The hair conditioning composition of any of the preceding claims, wherein the triester is a glycerol triester, wherein the glycerol triester is selected from the group consisting of trioctanoin, caprylic/capric triglyceride, and glyceryl tri (2-ethylhexanoate).

8. The hair conditioning composition of any one of the preceding claims 1 to 6, wherein the triester is a combination of: a triester having non-branched hydrocarbon groups; and a triester having branched hydrocarbon group.

9. The hair conditioning composition of any one of the preceding claims 1-6 or 8, wherein the weight ratio of non-branched triester to branched triester is from 1:3 to 3:1.

10. The hair conditioning composition of any one of the preceding claims 1-6 or 8-9, wherein the non-branched triester is caprylic/capric triglyceride.

11. The hair conditioning composition of any one of the preceding claims 1-6 or 8-10, wherein the branched triester is glyceryl tri (2-ethylhexanoate).
